(19) Europäisches Patentamt
European Patent Office
Office européen des brevets

(11) Publication number: **0 373 195 B1**

(12) **EUROPEAN PATENT SPECIFICATION**

(45) Date of publication of patent specification: **15.06.94**

(51) Int. Cl.$^5$: **C12N 5/00**

(21) Application number: **89904310.3**

(22) Date of filing: **27.03.89**

(86) International application number:
**PCT/US89/01265**

(87) International publication number:
**WO 89/09258 (05.10.89 89/24)**

(54) **COMPOSITION FOR INCREASING MELANIN CONTENT OF MELANOCYTES IN-VIVO AND IN-VITRO AND METHODS FOR THE IN-VITRO USE THEREOF.**

(30) Priority: **30.03.88 US 175179**

(43) Date of publication of application:
**20.06.90 Bulletin 90/25**

(45) Publication of the grant of the patent:
**15.06.94 Bulletin 94/24**

(84) Designated Contracting States:
**BE DE FR GB IT SE**

(56) References cited:

**ANN. NY. ACAD. SCI., vol. 548, 1988, pages 180-190; R. HALABAN et al.**

**CHEMICAL ABSTRACTS, vol. 100, no. 1, 2nd January 1984, page 113, abstract no.1293t, Columbus, Ohio, US; B.A. GILCHREST et al.**

**Journal of Cellular Physiology, 122 (3), 1985, pp. 350-361, Wilkins et al.**

(73) Proprietor: **TRUSTEES OF BOSTON UNIVERSITY**
**881 Commonwealth Avenue**
**Boston, Massachusetts 02215(US)**

(72) Inventor: **GILCHREST, Barbara, A.**
**67 Walnut Place**
**Brookline, MA 02146(US)**
Inventor: **GORDON, Philip, R.**
**456 Centre Street, No. 3**
**Jamaica Plain, MA 02130(US)**

(74) Representative: **Eyles, Christopher Thomas et al**
**W.P. THOMPSON & CO.**
**Celcon House**
**289-293 High Holborn**
**London WC1V 7HU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

### FIELD OF THE INVENTION

The present invention is concerned generally with the cells of and the cellular reactions within the skin of living humans and animals and is particularly directed to novel compositions for increasing the melanin content of skin in living subjects in-vivo and in-vitro and to a method for the in-vitro use thereof.

### BACKGROUND OF THE INVENTION

Melanins are a class of structurally related compounds that serve as the principal pigments of mammalian skin and hair. Melanin pigmentation is largely responsible for normal skin color and hair color; and provides protection against ultraviolet light damage from sunlight. In the skin, melanins are synthesized exclusively by specialized cells termed "melanocytes" found in the skin and hair follicles. Once synthesized, melanin is transferred via the cellular dendrites of the melanocyte to the surrounding keratinocytes, the most abundant cell type in the epidermis. This anatomical relationship, often termed the epidermal melanin unit, is generally envisioned in-vivo as being one melanocyte in contact with an estimated 36 keratinocytes in the basal and suprabasal layers of the epidermis. The rates of melanin pigment synthesis and the subsequent transfer of melanin by melanocytes appear to be influenced by ultraviolet light exposure and by certain inflammatory processes. The net result can take a number of different forms, some of which are considered normal and some of which are considered pathological.

Darker skin pigmentation is considered desirable by many persons, socially and esthetically. In addition, a high melanin content in human skin has recognized medical value. Unfortunately, the only presently known means of darkening skin is sun-tanning using either natural sunlight or specially designed ultraviolet light sources (tanning lamps). Exposure of human skin is well known to have adverse long term and short term health consequences, specifically skin cancer and photoaging (long term) and the risk of painful sunburn and keratitis (short term). Light-skinned individuals are highly susceptible to sun-induced skin cancers; face a high risk of melanoma and carcinoma; and incur photoaging or dermatoheliosis, a condition characterized by wrinkling, irregular pigmentation, and surface roughness. However, even darker skinned individuals exposed to prolonged sunlight incur a high risk of skin cancer and unwanted aging changes.

Other individuals are unable to achieve even normal pigmentation due to their being afflicted with vitiligo, piebaldism, albinism, or other hypopigmentation disorders. The result of such abnormal conditions, in the extreme, is total depigmentation of both hair and skin. In less severe instances, some hypopigmentation disorders result in patchy white areas within the skin and hair. All of these conditions cause severe cosmetic problems and mental stress.

Melanocytes are also responsible for the pigmentation of hair and feathers. Graying of hair is due to a decrease in number or activity of the melanocytes residing in the hair bulb. In non-human instances, changes in melanocyte content and melanin synthesis rate result in changes in the color of pelage, fur, wool, and other kinds of animal hair; the ability to maintain melanin production would thus prevent discoloration in fur, wool, feathers, and other animal hair counterparts. Controlling pigment synthesis in animals would permit also production of biologically engineered fur, wool, and feathers.

With the evolution of tissue culture techniques in recent years, a variety of different research efforts and investigations utilizing in-vitro tissue culture methods have been undertaken. Many reports and publications now exist in the scientific and technological literature directed to morphological and biochemical examination of the cells within the skin and the mechanism of their interactions as these relate to the growth of melanocytes and the production of melanin. These publications and reports can be separated into four broad categories of investigations and information: First, in-vitro culture media and cell culture techniques for the growth and maintenance of keratinocytes and melanocytes. This category is represented by the following publications: Gilchrest and Friedmann, Structure And Function Of Melanin (Jimbow, K., editor), Fuji-shoin Company, Ltd., Sapporo, Japan, Volume 4, 1978, pages 1-13; Gilchrest et al., J. Cell. Physiol. 117:235-240 (1983); Gilchrest et al., J. Invest. Dermatol. 83:370-376 (1984); U.S. Patent Number 4,443,546; U.S. Patent Number 4,507,321; U.S. Patent Number 4,456,687; U.S. Patent Number 4,444,760; Gilchrest et al., J. Cell. Physiol. 112:197 (1982); Groelke et al., In-vitro 17:247 (1981); Maciag et al., Science 211:1432 (1981); Rheinwald et al., Nature 265:421 (1977); and Rheinwald et al., Cell 6:331 (1975).

The second broad category includes investigations into simulating agents and mitogens for melanocytes. A representative listing of publications in this second category include the following: Gordon et al., J. Invest. Dermatol. 87:723-727 (1986); Wilkins et al., J. Cell. Physiol. 122:350-361 (1985); Levine et al., J. Invest. Dermatol. 89:269-273 (1987); International Application Number PCT/US87/00226 published as

International Publication Number WO87/04623 on 13 August 1987; Ogata et al., Biochem. Biophys. Res. Comm. 146:1204-1211 (1987); Halaban et al., In-vitro 23:47-52 (1987); Bregman et al., Exp. Cell. Res. 157:419-428 (1985); Nordlund et al., J. Invest. Dermatol. 86:433-437 (1986); Hadley et al., Endoc. Res. 11:157-170 (1985); and Preston et al., Proc. Natl. Acad. Sci. USA 84:5247-5251 (1987).

The third broad category of publications is directed to identifying the biochemical events, the chemical entities, and the intracellular pathways and mechanism of action within melanocytes under varying conditions. Publications representative of this broad category include the following: Hadley et al., "Biological Actions of Melanocyte-Stimulating Hormone," in Peptides Of The Pars Intermedia, Pitman Medical Company, London, 1981, pages 244-262; Lerner and McGuire, N.E.J. Med. 270:539-546 (1964); Friedmann and Gilchrest, J. Cell Physiol. 133:88-94 (1987); Nordlund, J.J., Dermat. Clin. 4:407-418 (1986); Nordlund and Abdel-Malek, Prog. Clin. Biol. Res. 256:219-236 (1988); Castagna, M., Biol. Cell 59:3-14 (1987); Abdel-Malek et al., In-vitro 22:75-81 (1986); Rosen et al., J. Invest. Dermatol. 88:774-779 (1987); Halaben et al., Arch. Biochem. Biophys. 230:383-387 (1984); and Preston et al., Proc. Natl. Acad. Sci. USA 84:5247-5251 (1987).

The fourth broad category is directed to techniques and methods for directly inducing or increasing melanin production by melanocytes. This fourth category is the least developed and investigators have often resorted to the use of murine skin and murine melanoma cell lines rather than using human melanocytes. Representative of this fourth broad category are the following publications: Friedmann and Gilchrest, J. Cell. Physiol. 133:88-94 (1987); Rosdahl and Szabo, J. Invest. Dermatol. 70:143-148 (1978); Warren, R., Photochem. Photobiol. 43:41-47 (1986); Jimbow and Uesugi, J. Invest. Dermatol. 78:108-115 (1982); Rosen et al., J. Invest. Dermatol. 84:5247-5251 (1987); U.S. Patent Number 4,508,706; U.S. Patent Number 4,618,484; and U.S. Patent Number 4,695,449.

Because of the variety and the scope of the pertinent literature in general, a summary of the pertinent facts believed to be true and supported by empirical evidence can be stated as follows: pure cultures of melanocytes respond directly to simulated sunlight by an increased melanin content per cell; by morphological changes; and by cellular growth arrest. These responses are recognized as partially mimicking the tanning response of melanocytes in intact skin. This in-vitro tanning response is not attributed to an altered cAMP content or to the effects of vitamin D, its precursors, photoproducts, or metabolites. In addition, it has been demonstrated that a variety of different tissue extracts and derived growth factors can stimulate melanocyte proliferation However, the use of many stimulating agents and mitogens such as choleragen and phorbol esters, although inducing cell proliferation, also render the melanocytes insensitive to many other physiologic stimuli. Moreover, as part of the response to most mitogens in-vitro, there is an inverse relationship between melanocyte growth rate and melanin content per cell. This is in contrast to living skin which responds to ultraviolet irradiation with both an increase in the number of melanocytes and an increase in the melanin synthesis rate per melanocyte. Finally, while the relationship between human keratinocytes and melanocytes continues to be a subject of considerable interest, there remains a noticeable absence of information and empirical data regarding what, if any, soluble keratinocyte cellular products enhance or influence melanocyte growth rate, melanocyte dendricity, and/or melanin pigment production by melanocytes. It would be recognized and appreciated, therefore, by practitioners ordinarily skilled in this art, that the availability of specific in-vitro culture medium products generated by keratinocytes that can individually affect and control melanocyte proliferation, or melanocyte dendricity, or melanin synthesis by melanocytes would be a major advance and recognized improvement over all presently known methods and compositions.

## SUMMARY OF THE INVENTION

The present invention provides a method for increasing the melanin content of melanocytes, in-vitro, said method comprising the steps of:

obtaining a diacylglycerol; and

adding said diacylglycerol to the melanocytes.

It further provides a topical formulation for increasing the melanin content of the skin in-vivo, said formulation comprising:

a fluid carrier compatible with the skin of a living subject; and

a diacylglycerol.

A material useful in the composition and method of the invention is at least one member selected from the chemical class consisting of diacylglycerols, diacylglycerol analogues, and diacylglycerol derivatives.

Each or any of these diacylglycerols can be combined in admixture with a fluid carrier compatible with the skin of a living subject to form a topical formulation able to increase the melanin content of skin in-vivo. These unique compositions may also be employed with conventionally known media and culture techniques

for in-vitro culture of melanocytes whether for scientific research or commercial purposes. In each instance, the use of these compositions in-vivo and in-vitro will increase melanin synthesis within melanocytes.

## BRIEF DESCRIPTION OF THE FIGURES

The present invention may be more easily and completely understood when taken in conjunction with the accompanying drawing, in which:

Fig. 1a and 1b are graphs illustrating the effects of a diacylglycerol upon human melanocyte proliferation and melanin content;

Fig. 2 is a graph illustrating the effects of a diacylglycerol upon the proliferation of human melanocytes when administered alone and after pretreatment with a phorbol ester;

Fig. 3 is a graph illustrating the effects of a diacylglycerol upon melanin production with human melanocytes when administered alone and after pretreatment with a phorbol ester;

Fig. 4 is a graph illustrating the effects of a diacylglycerol on the proliferation of S91 melanoma cells in culture; and

Fig. 5 is a graph illustrating the effects of a diacylglycerol upon the melanin content of S91 melanoma cells in culture.

## DETAILED DESCRIPTION OF THE PREFERRED EMBODIMENTS

The present invention relates to a general methodology for controlling the biological activity of human and animal melanocytes under in-vitro conditions. It further relates to topical formulations for controlling the biological activity of human and animal melanocytes under in-vivo conditions. The present invention, in all its aspects, is based and relies upon a fundamental premise that the ability to control and specifically regulate melanocyte growth and melanocyte dendricity is separately controllable and distinguishable from the ability to regulate the rate of melanin synthesis in the melanocyte. The premise is, therefore, that several different factors and mediators regulate and modulate each of these specific activities independently, but in concert with the others. Thus, for example, while the rate of melanin synthesis is directly controlled by one or more specific factors or mediators, there will be some degree of interaction and cross-effects with the other specific factors or mediators individually responsible for regulation of melanocyte growth and melanocyte dendricity. This fundamental premise differs substantially from the conventional approach.

The conventional outlook documented and accepted by ordinary practitioners in this art follows and complies with traditional histological studies. In contrast a novel sequence of events occurring in primary in-vitro cultures of human epidermis have been observed between the two relevant types of cells, keratinocytes and melanocytes. Initially, the epidermal suspension maintained in nutritionally adequate culture medium contains more than 90% keratinocytes and only an estimated 2-4% melanocytes. In some culture systems, the culture medium is such that the keratinocyte population dwindles and is lost within a few days, thereby prohibiting any meaningful interaction with melanocytes. In other culture systems, the keratinocytes survive but the proportions of the cells gradually reverse after two or three weeks culture as the melanocytes proliferate and the keratinocytes terminally differentiate in a selective growth medium. At this time, a striking preferential clustering of dendritic melanocytes around the few remaining viable keratinocytes is readily apparent. Complete disappearance of keratinocytes from the cultures occurs after three or four weeks; and is accompanied by cessation of melanocyte proliferation, and a decrease in melanocyte dendricity with substantial alteration of these cells to a polygonal cell morphology. To our knowledge, there has been no common or generally accepted belief heretofore that a regulatory role for keratinocytes might exist in determining melanocyte morphology, growth, or melanization. Hence, even the concept that keratinocytes play a major role is new; and the premise that different soluble factors and/or mediators generated and released by keratinocytes in culture could specifically regulate individual parts of the process separately or in concert is entirely novel and unforeseen in view of existing knowledge.

There is also a second major difference and distinction in fundamental premise and approach of the present invention from the conventionally held views and beliefs of practitioners in this art. A substantial portion of the published literature describing both in-vivo and in-vitro experiments and providing empirical data have utilized non-human, animal models and employed non-human keratinocytes and melanocytes. A favorite of many investigators is the use of mouse cells, the S91 mouse melanoma cells being perhaps the best known. The prevalent view is that the animal data and models are directly applicable to the human melanocyte condition; and that the stimulating agents, mitogens, and other mediators of melanocyte activity effective within the mouse and other animal cell systems are also operative and in existence for the human

melanocyte and human skin. The present invention does not accept this view and in fact provides evidence that specific factors and intracellular pathways mediate pigmentation in human skin which are different from those present in animal skin. The factors that govern human melanocyte growth, human pigment production, and human pigment transfer are effective regulatory controls for human melanocytes; whereas factors and mediators derived from non-human keratinocytes are often ineffective and non-regulatory. The second fundamental difference is thus the realization that major differences and distinctions exist among the factors and mediators of melanocytes effective in human and non-human cellular conditions.

The present invention provides a class of diacylglycerol compositions, analogues, and derivatives, which can be usefully employed in the methodology for a variety of different purposes and advantages.

The class of diacylglycerol compositions is particularly useful for humans and provides the following benefits and advantages:

(a) inducing tanning of the skin in the absence or presence of sunlight;

(b) acting as an accelerator of skin tanning in the presence of natural sunlight; and

(c) providing a treatment for gray (depigmented) hair.

The chemical class of diacylglycerols as a whole are materials which interact with living melanocytes, in-vivo or in-vitro.

Keratinocytes and Melanocytes

The source for both kinds of human cells is preferably neonatal foreskins obtained within two hours of elective circumcision. The epidermis is separated from the dermis after overnight incubation in 0.25% trypsin [GIBCO, Grand Island, New York; Gilchrest et al., J. Invest. Dermatol. 83:370-376 (1984)]. Primary cultures of epidermal keratinocytes are established using the method of Rheinwald and Green [Cell 6:331-334 (1975)], modified by using a 4:1 ratio of Dulbecco's modified Eagle's medium (hereinafter "DMEM") and Ham's F12 medium supplemented with 10 mM adenine and 10% fetal bovine serum [MA Bioproducts, Walkersfield, MR].

The confluent primary cultures of keratinocytes are then washed with 0.5 mM ethylenediamine tetracetic acid (hereinafter "EDTA") and incubated with 1 milliliter (hereinafter "ml") of 0.25% trypsin for approximately 15 minutes. The resulting cell suspension is then mixed with an equal volume of 10% FBS in M199 culture medium [GIBCO]. The cells were then pelleted by centrifugation; resuspended in BITIC medium; and seeded at 1 x $10^6$ cells into 60 millimeter (hereinafter "mm") dishes containing 4 ml of BITIC medium. BITIC medium is prepared as follows:

| BITIC MEDIUM (500 ml) | | | |
|---|---|---|---|
| | ADDxml | STOCK | FINAL |
| Medium 199 (Gibco 400-1100) | 491 | | |
| Insulin* (INS) | 0.5 | 10 mg/ml | 10 ug/ml |
| Epidermal Growth Factor (EGF) | 0.5 | 10 ug/ml | 10 ng/ml |
| Triiodothyronine (T3) | 0.5 | 1 uM | 1 nM |
| Transferrin (Tf) | 0.5 | 10 mg/ml | 10 ug/ml |
| Hydrocortisone (HC) | 0.5 | 1.4 mM | 1.4 uM |
| Inositol (Ino) | 0.5 | 10 mg/ml | 10 ug/ml |
| Choline Chloride (CC) | 0.5 | 10 mg/ml | 10 ug/ml |
| Bovine Serum Albumin (BSA) | 5 | 200 mg/ml | 2 mg/ml |
| Penicillin/Streptomycin** (P/S) (optional) | 5 | | 1% v/v |

* Insulin is added first as it is acidic

**If Pen/Strep is used, reduce M199 volume to 486.

Before plating, the culture dishes were pretreated with 30 micrograms (hereinafter "ug") per dish of affinity purified human fibronectin. The cultures were then incubated in 8% carbon dioxide/air at 37 C and provided with fresh medium twice weekly.

Melanocytes were established in primary culture from similarly prepared epidermis following the procedure of Gilchrest et al. [J. Invest. Dermatol. 83:370-376 (1984)]. In brief, operative specimens were cut into 4 $mm^2$ fragments; rinsed in calcium-free phosphate-buffered saline (hereinafter "PBS"); and incubated in 0.25% trypsin (GIBCO) overnight at 4 C. The epidermal portions of the fragments were separated from the dermis with forceps; incubated for 10 minutes in 0.02% EDTA at 37 C; vortexed to yield a single cell

suspension; inoculated at a concentration of $10^6$ cells per 35-mm dish in melanocyte growth medium; and maintained at 37 C in 8% carbon dioxide and 92% air. Cultures were provided with fresh melanocyte growth medium three times weekly.

Melanocyte growth medium is prepared as follows:

| MELANOCYTE GROWTH MEDIUM (500 ml) | | | |
|---|---|---|---|
| | ADDxml | STOCK | FINAL |
| Medium 199 (Gibco 400-1100) | 467 | | |
| Insulin* (INS) | 0.5 | 10 mg/ml | 10 ug/ml |
| Triiodothyronine (T3) | 0.5 | 1 µM | 1 nM |
| Epidermal Growth Factor (EGF) | 0.5 | 10 µg/ml | 10 ng/ml |
| Transferrin (Tf) | 0.5 | 10 mg/ml | 10 ug/ml |
| Hydrocortisone (HC) | 0.5 | 1.4 mM | 1.4 µM |
| Cholera Toxin (CT) | 5 | $10^{-7}$ M | $10^{-9}$ M |
| dialyzed Brain Extract (dBE) | 25 | 2 mg/ml | 100 µg/ml |
| Penicillin/Streptomycin** (P/S) (optional) | 5 | | 1% v/v |

* Insulin is added first as it is acidic.

** If Pen/Strep is used, reduce M199 volume to 462.

Regarding keratinocytes and melanocytes from non-human sources, it is expected that the origin of the cells and the manner in which each is isolated and maintained in culture will follow those procedures previously established in the literature for these specific purposes.

Keratinocyte Product Generating Medium (KPGM)

It is intended that a variety of different formulations and compositions in admixture can be utilized as KPGM for purposes of the present invention. KPGM is thus not a single formulation or mixture of specific ingredients, but includes a variety of differently prepared culture media suitable for in-vitro maintenance of grown keratinocytes for the specific purpose and goal of inducing the keratinocytes in culture to generate and release a variety of different factors and/or mediators as products into the surrounding medium. KPGM therefore may be most broadly defined as a variety of specifically prepared maintenance media for continuing in-vitro culture of grown keratinocytes. ▲ At a minimum, this medium comprising the following: at least an isotonic salt solution at about neutral pH values for minimal maintenance of grown keratinocytes in culture;

0 - 20 mg/ml albumin;
0 - 100 µg/ml inositol; and
0 - 50 µg/ml choline chloride.

▲ An alternative minimal formulation will comprise the following:
essential amino acids, vitamins, nutrients, and salts for minimal maintenance of keratinocytes in culture;
1 - 100 µg/ml insulin;
0 - 10 nM of a iodothyronine, preferably triiodothyronine;
0 - 100 µg/ml transferrin;
0 - 15 M of a cortisone, preferably hydrocortisone; and
0 - 100 ng/ml epidermal growth factor.

▲ A preferred Formula I for KPGM comprises:
Dulbecco's Minimal Eagle's medium containing:
10 µg/ml insulin;
1 nM triiodothyronine;
10 µg/ml transferrin;
1.4 x $10^{-6}$ M hydrocortisone;
10 ng/ml epidermal growth factor; and
$10^{-9}$ M choleragen (chlorea toxin).

▲ An alternate preferred Formula II for KPGM comprises:
Medium 199 (GIBCO) containing 0.3 µM myo-inositol, 3.6 uM choline, 1.8 µM calcium, and 0.5 mM chloride;
10 µg/ml insulin;

$10^{-9}$ M transferrin;
1.4 x $10^{-6}$ M hydrocortisone;
10 ng/ml epidermal growth factor; and
2 mg/ml bovine serum albumin.

▲ An alternate preferred Formula III for KPGM includes the following:
1 part Dulbucco's Minimal Eagle's medium;
4 parts Ham's F12 medium;
10 mM adenine;
10% fetal calf serum;
10 µg/ml insulin;
$10^{-9}$ M triiodothyronine;
10 µg/ml transferrin;
1.4 x $10^{-6}$ M hydrocortisone; and
10 ng/ml epidermal growth factor.

▲ Within these alternative definitions of KPGM, a number of additives may be optionally added for increasing the longevity of the keratinocytes in culture or increasing the concentration of soluble factors and/or mediators released into the medium during keratinocyte culture. These include:

20 - 300 µg/ml of bovine hypothalmic extract (Gilchrest et al., J. Invest. Dermatol. 83:370-376 (1984); Wilkins et al., J. Cell. Physiol. 122:350-361 (1985)];
0.5 - 500 µM myo-inositol;
0.4 - 40 µM choline and 0.5 - 500 µM myo-inositol in combination;
0.5 - 500 µM ethanolamine;
0.5 - 500 µM phosphoethanolamine;
0 - 5 mM serine;
0 - 1 uM selenium;
0 - 10 uM prostaglandin $E_2$, $E_1$; and
$10^{-8}$ - $10^{-13}$ M cholera toxin.

▲ Compounds to be avoided as additives to KPGM generally include the following:
stearic acid in either dilute or concentrated form;
methionine in either dilute or concentrated form;
cyanocobalamine in either dilute or concentrated form; and
folic acid in either dilute or concentrated form.

## Culture Conditions for KPGM An Keratinocytes In-Vitro

The incubation conditons for keratinocytes and the KPGM follow conventional routine. All cultures are preferably incubated at 37°C ±4°C in an atmosphere containing 8 ±4% carbon dioxide. Fresh medium is added to each dish at intervals ranging from 1 to 4 times per week. Use of sterile technique and sterile precautions is presumed throughout.

## DIACYLGLYCEROLS (DAG)

### Chemical Structure and Occurrence

Diacylglycerols (hereinafter "DAG") are a class of organic chemical compositions having the chemical structure and formulation of Compounds A, B, or C as follows:

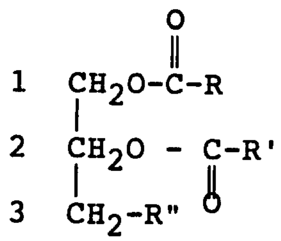

(A)

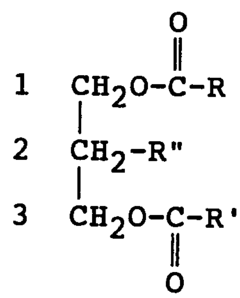

(B)

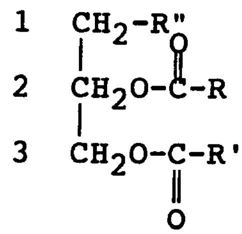

(C)

in which R and R' are carbon containing moieties. R and R' are usually long-chain (greater than 14 carbon atoms) carboxylic acids and may contain one or more carbon-carbon double bonds. In most instances, R and R' are chemically different in composition. In comparison, R" can be any chemical entity which does not form a carbon ester linkage with the adjoining carbon atom in the glycerol structure. Accordingly, R" may include a hydroxyl group, a phosphate group, a sulfur atom, an ether group, a halide, a nitrogen containing entity, or hydrogen.

Diacylglycerols are a class of naturally occurring substances. One known role for diacylglycerols is in an intracellular signal transduction pathway in which DAGs are recognized as physiological activators of protein kinase C (hereinafter "PKC"); and phorbol esters can act through similar biochemical pathways [Berridge, N.J. Ann. Rev. Biochem. 56:159-193 (1987); Nishizuka, Y., Science 233:305-312 (1986)]. The synthesis of DAG and many DAG analogues is conventionally known [Ganong and Bell, Methods In Enzymology, Volume 141, 1987, pages 313-320].

Diacylglycerols Of The Present Invention

The diacylglycerols of the present invention are those DAG compositions and analogues which are biologically active to produce an increase in cellular melanin content in melanocytes under in-vivo and/or in-vitro conditions. The naturally occurring DAGs are derivatives of phosphatidylinositol; usually contain a long-chain mono-unsaturated fatty acid acylated to the number one carbon position in the glycerol structure; and also typically contain a highly unsaturated fatty acid, primarily arachidonic acid, acylated to the number two carbon position in the glycerol structure. The most preferred member of this chemical class for use in the present methodology is 1-oleoyl-2-acetyl-glycerol because of its solubility in water and its ability to produce a dose-dependent response in melanin content with no concommitant effect upon proliferation and/or growth of melanocytes. In addition, other preferred embodiments for DAG contain a free hydroxyl group at the number three carbon position in the glycerol structure; and are limited to the traditional three carbon backbone structure of glycerol. A representative, but incomplete listing of DAG molecules believed to be biologically active and potent for stimulation of melanin synthesis within melanocytes are those provided by Table II below.

PREFERRED DAG ANALOGUES

1,2-diformylglycerol
1,2-diacetylglycerol
1,2-dibutanoylglycerol
1,2-dihexanoylglycerol
1,2-dioctanoylglycerol
1,2-didecanoylglycerol
1,2-didodecanoylglycerol
1,2-ditetradecanoylglycerol
1,2-dihexadecanoylglycerol
1,2-dioctadecanoylglycerol
1,2-dieicosanoylglycerol
1,2-didocosanoylglycerol
1,2-ditetracosanoylglycerol
1,2-dipalmitoylglycerol
1,2-dioleoylglycerol
1,2-dilinoleoylglycerol
1,2-dilinolenoylglycerol
1,2-arachidonoylglycerol
1-octanoyl-2-formyl-glycerol
1-octanoyl-2-acetyl-glycerol
1-octanoyl-2-butanoyl-glycerol
1-octanoyl-2-hexanoyl-glycerol
1-octanoyl-2-decanoyl-glycerol
1-octanoyl-2-dodecanoyl-glycerol
1-octanoyl-2-tetradecanoyl-glycerol
1-octanoyl-2-hexadecanoyl-glycerol
1-octanoyl-2-octadecanoyl-glycerol
1-octanoyl-2-eicosanoyl-glycerol
1-octanoyl-2-docosanoyl-glycerol
1-octanoyl-2-tetracosanoyl-glycerol
1-octanoyl-2-palmitoyl-glycerol
1-octanoyl-2-oleoyl-glycerol
1-palmitoyl-2-formyl-glycerol

PREFERRED DAG ANALOGUES (CONT'D)

1-palmitoyl-2-acetyl-glycerol
1-palmitoyl-2-butanoyl-glycerol
1-palmitoyl-2-hexanoyl-glycerol
1-palmitoyl-2-octanoyl-glycerol
1-palmitoyl-2-decanoyl-glycerol
1-palmitoyl-2-dodecanoyl-glycerol
1-palmitoyl-2-tetradecanoyl-glycerol
1-palmitoyl-2-hexadecanoyl-glycerol
1-palmitoyl-2-octadecanoyl-glycerol
1-palmitoyl-2-eicosanoyl-glycerol
1-palmitoyl-2-docosanoyl-glycerol
1-palmitoyl-2-oleoyl-glycerol
1-palmitoyl-2-linoleoyl-glycerol
1-palmitoyl-2-arachidonoyl-glycerol
1-oleoyl-2-formyl-glycerol
1-oleoyl-2-acetyl-glycerol
1-oleoyl-2-butanoyl-glycerol
1-oleoyl-2-hexanoyl-glycerol
1-oleoyl-2-octanoyl-glycerol
1-oleoyl-2-decanoyl-glycerol
1-oleoyl-2-dodecanoyl-glycerol
1-oleoyl-2-tetradecanoyl-glycerol
1-oleoyl-2-palmitoyl-glycerol
1-oleoyl-2-linoleoyl-glycerol
1-oleoyl-2-arachidonoyl-glycerol
1-hexanoyl-2-formyl-glycerol
1-hexanoyl-2-acetyl-glycerol
1-hexanoyl-2-butanoyl-glycerol
1-hexanoyl-2-octanoyl-glycerol
1-hexanoyl-2-decanoyl-glycerol
1-hexanoyl-2-dodecanoyl-glycerol
1-hexanoyl-2-tetradecanoyl-glycerol
1-hexanoyl-2-hexadecanoyl-glycerol

PREFERRED DAG ANALOGUES (CONT'D)

1-hexanoyl-2-octadecanoyl-glycerol
1-hexanoyl-2-eicosanoyl-glycerol
1-hexanoyl-2-palmitoyl-glycerol
1-hexanoyl-2-oleoyl-glycerol
1-hexanoyl-2-linoleoyl-glycerol
1-hexanoyl-2-arachidonoyl-glycerol

Diacylglycerol Administration and Manner of Use

As will be empirically demonstrated hereinafter, DAG analogues are able to produce increases in cellular melanin content without altering melanocyte proliferation. Moreover, DAG analogues do not increase dendricity of melanocytes. DAGs are therefore selective to their biological action and cellular function; and are generally limited to being able to induce melanin synthesis and production specifically within preexisting melanocytes.

It is intended that the DAG analogues be employed within in-vivo and in-vitro conditions. For in-vivo use, it is desirable that topical administration of one or more DAG analogues be made directly to the skin of the living subject. For this purpose, a fluid carrier is intended to be combined in admixture with the chosen DAG analogue. Accordingly, the DAG analogues are intended to be admixed in a pharmacological topical carrier such as a gel, an ointment, a lotion, or a cream; and will include such fluid carriers as water, glycerol, alcohol, propylene glycol, fatty alcohols, triglycerides, fatty acid esters, or mineral oils. Other possible topical carriers are liquid petrolatum, isopropyl palmitate, polyethylene glycol, ethanol (95%), polyoxyethylene monolaurate (5%) in water, sodium lauryl sulfate (5%) in water, and the like. Other materials such as anti-oxidants, humectants, viscosity stabilizers, and similar agents may be added as necessary. In addition, in certain instances, it is expected that the supplemented preparations as described herein may be disposed within devices placed upon, in, or under the skin; such devices include patches, implants, and injections which release the supplemented preparation into the skin either by passive or active release mechanisms. For in-vivo use, it is preferred that the DAG analogue be present in a final concentration range from 0.10 - 20.00 mM in a fluid carrier material. Alternatively, for in-vitro use as a laboratory reagent with cultures of melanocytes, the chosen DAG analogue may be added directly to the culture media surrounding the living cells at any desired concentration in accordance with the purposes and goals of the experimental design. It is clearly expected and intended that empirical determinations of inhibitory and toxic concentrations for the chosen DAG analogue will be made; accordingly, there is no effective limit on the concentration of the chosen DAG analogue within these in-vitro applications and uses.

As will be empirically demonstrated hereinafter, the preferred DAG analogue, 1-oleoyl-2-acetyl-glycerol is selective in its activity with melanocytes from human skin; in contrast, this DAG analogue is demonstratably inactive and does not induce increases in melanin production when administered to S91 mouse melanoma cells, the most commonly tested animal melanocyte model.

To demonstrate the unique biological activity and operability of the compositions and method comprising the present invention as a whole, a variety of different experiments performed in the laboratory will be described. These in-vitro experiments serve merely to illustrate the diversity of applications and conditions with which the methods of the present invention may be usefully employed. While the experimental design is solely in laboratory scale terms, it is clear that the empirical parameters can be expanded at will to meet different in-vivo and in-vitro applications. Moreover, the described empirical data and experiments will be clearly understood to be only representative of the number, variety, and diversity of compositions and culture media which can be advantageously employed.

Melanocyte Bioassay

Melanocytes were seeded at 2 x $10^4$ cells per 35 mm dish and combined with KPGM supplemented with 2% FBS and 100 ug/ml of BHE, now designated as complete melanocyte medium. After 24 hours incubation at 37 C, the grown melanocyte cultures, in duplicate or triplicate, received one of the following: free complete melanocyte medium; or supplemented whole KDCM. Each melanocyte culture was then incubated for 6-7 days at 37 C. Subsequently, each melanocyte culture was harvested and the cell number per dish empirically determined. Each melanocyte culture was washed with a 0.4 mM EDTA in PBS; treated with 1 ml of a mixture containing 0.13% trypsin and 0.2 mM EDTA; then incubated approximately 10 minutes at 37 C; followed by addition of 1 ml of PBS. Subsequently, a 0.5 ml aliquot of each resulting suspension was diluted to 10 ml total volume using isotonic saline and processed using a particle counter (Model ZM, Colter Science).

To determine melanin content, the remaining suspension was centrifuged for 5 minutes in a microcentrifuge; the supernatant discarded; and the resulting cell pellet dissolved in 0.1 ml of a 1 M NaOH which was subsequently diluted with 0.4 ml of water. Melanin concentration was calculated by determination of optical density at 475 nanometers; and values extrapolated by comparison with a standard curve of determinations for synthetic melanin, a measurement of melanogenesis which correlates extremely well with $^{14}$C-DOPA incorporation and with tyrosinase activity [Friedmann and Gilchrest, J. Cell. Physiol. 133:88-94 (1987)]. Melanin values were expressed as total melanin per culture; or as melanin content per cell; or as percent of untreated controls.

In certain instances, phase contrast micrographs were taken using an inverted microscope after the cultures were washed once with phosphate buffered saline.

Experiment 1: Effects of Diacylglycerols

This experimental series investigates the biological effects of diacylglycerols (DAG) upon human melanocytes and reveals the effects of pretreatment of human melanocytes with specific agents prior to incubation with a DAG. A first experimental series was performed which utilized human melanocytes combined with the preferred DAG, 1-oleoyl-2-acetyl-glycerol, selected for its efficiency of intercalating into melanocyte membranes. Neonatal foreskins obtained within two hours of elective circumcision were the source of human melanocytes. The epidermis was separated from the dermis after overnight incubation in 0.25% trypsin. Melanocytes were established in primary culture according to the procedure of Gilchrest et al. [J. Invest. Dermatol. 83:370-376 (1984)]. The preferred DAG was combined with human melanocytes in complete melanocyte medium and incubated together for a period of 6 days at 37°C. The results are graphically illustrated by Figs. 1a and 1b which evidence a clear dose dependent response to increased melanin content at concentration levels ranging from 25 - 200 μM with no meaningful effects on melanocyte proliferation or growth. At an optimal concentration of 100 μM, this DAG produced an average of a 4 fold increase in cellular melanin content per cell over the untreated control. It will also be noted that this DAG did not increase the dendricity of human melanocytes and did not have a meaningful effect upon human melanocyte growth.

A second experimental series was conducted in which the cultured human melanocytes were first pretreated with tetradecanoyl phorbol-13-acetate (hereinafter "TPA") at a concentration of 100 nM. Initially, the human melanocytes were obtained and grown as previously described. Subsequently, the cultured human melanocytes were combined with 100 nM of TPA in melanocyte medium and incubated at 37°C for 24 hours. The DAG was then added and the culture incubated again for 6 days. The melanin content of the cells was then evaluated in accordance with the procedure as previously described herein. The results are graphically illustrated by Figs. 2 and 3 respectively.

TPA alone induced extreme dendricity and diacylglycerol induced increased melanization. The combination was equally effective at melanization and the induction of dendricity. Such combinations would be able to increase both synthesis and pigment transfer.

Fig. 2 empirically demonstrated that neither the DAG analogue, nor the pretreatment with TPA, nor their combination has any substantial effect on human melanocyte proliferation. In comparison, Fig. 3 empirically demonstrates major and significant increases in melanin content per human melanocyte as a function of TPA pretreatment in amounts meaningfully greater than that of the chosen DAG analogue alone. Clearly, for maximum melanin production, it is most desirable to pretreat human melanocytes prior to administration of the chosen DAG analogue.

Experiment 2: Effects Of Diacylglycerol Analogues On Non-Human Melanocytes

This experimental series was performed to evaluate the effect of a DAG on melanocytes derived from animal sources, rather than humans. In this experimental series, murine S91 melanoma cells were grown and maintained in culture using conventional techniques [Friedmann and Gilchrest, J. Cell. Physiol. 133:88-94 (1987)]. The grown S91 melanoma cells were then combined with 100 µM of the preferred DAG, 1-oleoyl-2-acetyl-glycerol; or with 100 µM of 3-isobutyl-1-methylxanthine (IBMX). Each chemical agent was combined with the S91 melanoma cells in Dulbecco's Minimal Eagle's Medium containing 2% FBS at 37°C for 7 days. The results are graphically illustrated by Figs. 4 and 5 respectively.

Fig. 4 empirically demonstrates that the chosen DAG at concentrations ranging from 1-200 uM effectively failed to induce proliferation of S91 melanoma cells; the IBMX was similar in effect and also failed to produce any cellular growth. In comparison, Fig. 5 reveals major differences between the chosen DAG and IBMX to induce increases in melanin production. Clearly, IBMX at a concentration of 100 µM caused a major increase in melanin production in S91 melanoma cells; in comparison, the chosen DAG consistently failed to induce any substantial increases in melanin production in comparison to controls. It is thus unequivocally demonstrated that the biological effects of DAG are selective and discriminatory in accordance with the source of origin for the melanocytes. It is incontrovertible that this DAG has no meaningful effect on S91 melanoma cells, cells derived from mice; on the other hand, administration of this DAG to human melanocytes clearly and unequivocally causes major substantial increases in melanin content without concomitant cell proliferation of human melanocytes.

The present invention is not to be restricted in form nor limited in scope except by the claims appended hereto.

## Claims

1. A method for increasing the melanin content of melanocytes, in-vitro, said method comprising the steps of:
   obtaining a diacylglycerol; and
   adding said diacylglycerol to the melanocytes.

2. A method according to claim 1, wherein said melanocytes are maintained in culture.

3. A method according to claim 1 or claim 2, wherein said melanocytes are within skin tissue to be used as a skin.

4. Use of a diacylglycerol for the production of a topical formulation for increasing the melanin content of melanocytes in the skin in-vivo.

## Patentansprüche

1. Verfahren zur Erhöhung des Melaningehalts von Melanocyten in vitro mit den Stufen der
   Verschaffung eines Diacylglycerins und
   Zugabe des genannten Diacylglycerins zu den Melanocyten.

2. Verfahren nach Anspruch 1, bei dem die genannten Melanocyten in Kultur gehalten werden.

3. Verfahren nach Anspruch 1 oder Anspruch 2, bei dem die genannten Melanocyten innerhalb von als eine Haut zu verwendendem Hautgewebe sind.

4. Verwendung eines Diacylglycerins für die Herstellung einer örtlichen Formulierung zur Erhöhung des Melaningehalts der Melanocyten in der Haut in vivo.

## Revendications

1. Procédé d'élévation de la teneur en mélanine de mélanocytes, in vitro, ledit procédé comprenant les étapes consistant à :
   obtenir un diacylglycérol et
   ajouter ledit diacylglycérol aux mélanocytes.

2. Procédé selon la revendication 1, dans lequel lesdits mélanocytes sont maintenus en culture.

3. Procédé selon la revendication 1 ou la revendication 2, dans lequel lesdits mélanocytes se trouvent à l'intérieur de tissu cutané à utiliser comme peau.

4. Utilisation d'un diacylglycérol pour la préparation d'une formulation topique destinée à élever la teneur en mélanine de la peau in vivo.

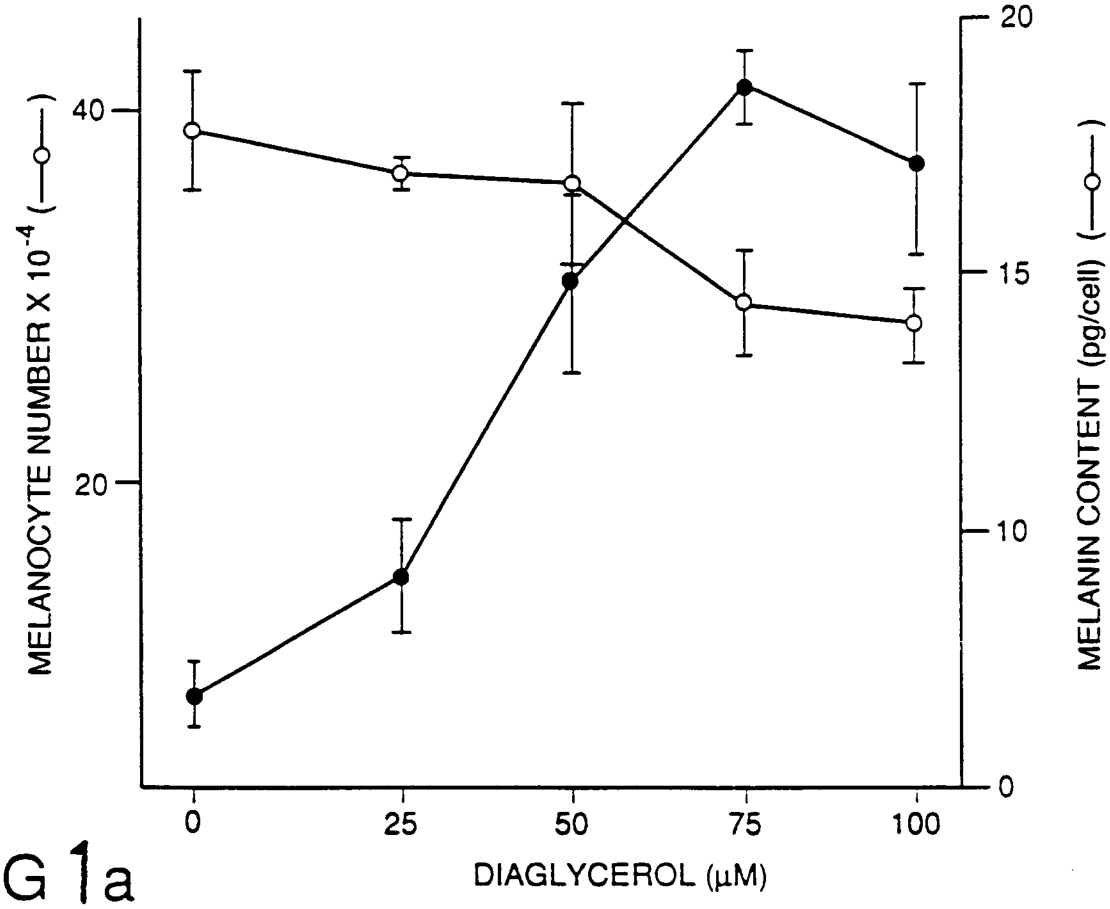
MELANOCYTE NUMBER X 10$^{-4}$
40
20
MELANIN CONTENT (pg/cell)
20
15
10
0
0
25
50
75
100
DIAGLYCEROL (μM)

FIG 1a

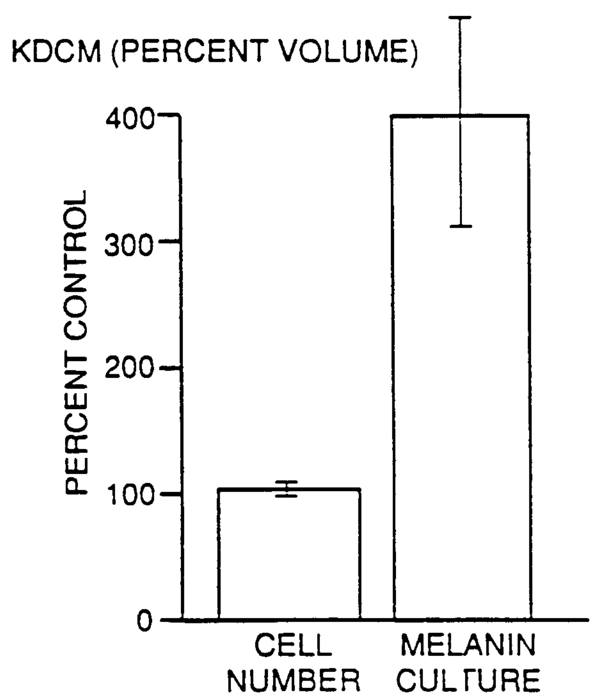
KDCM (PERCENT VOLUME)
PERCENT CONTROL
400
300
200
100
0
CELL NUMBER
MELANIN CULTURE

FIG 1b

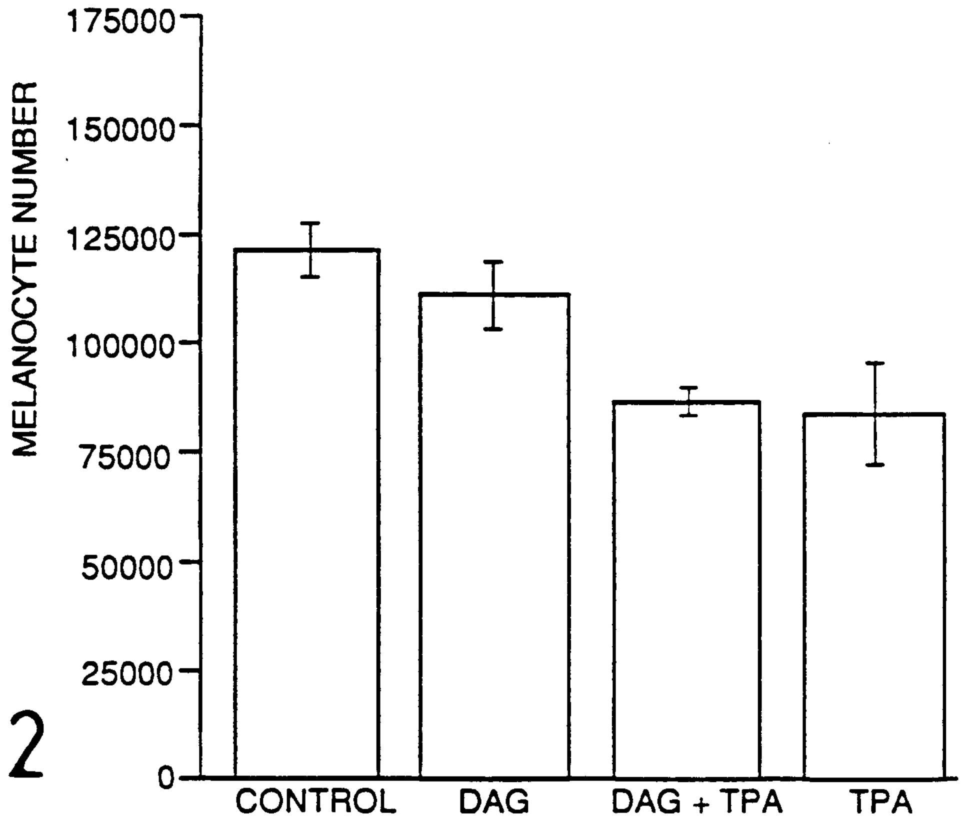
MELANOCYTE NUMBER
175000
150000
125000
100000
75000
50000
25000
0
CONTROL
DAG
DAG + TPA
TPA

FIG 2

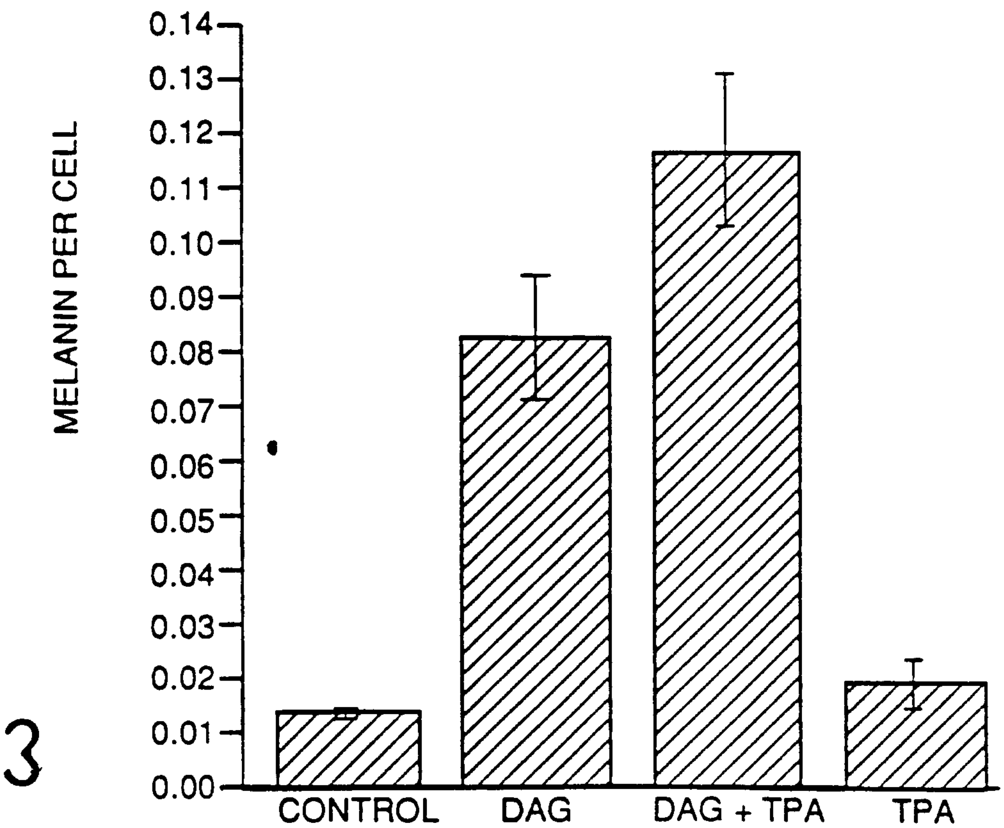
MELANIN PER CELL
0.14
0.13
0.12
0.11
0.10
0.09
0.08
0.07
0.06
0.05
0.04
0.03
0.02
0.01
0.00
CONTROL
DAG
DAG + TPA
TPA

FIG 3

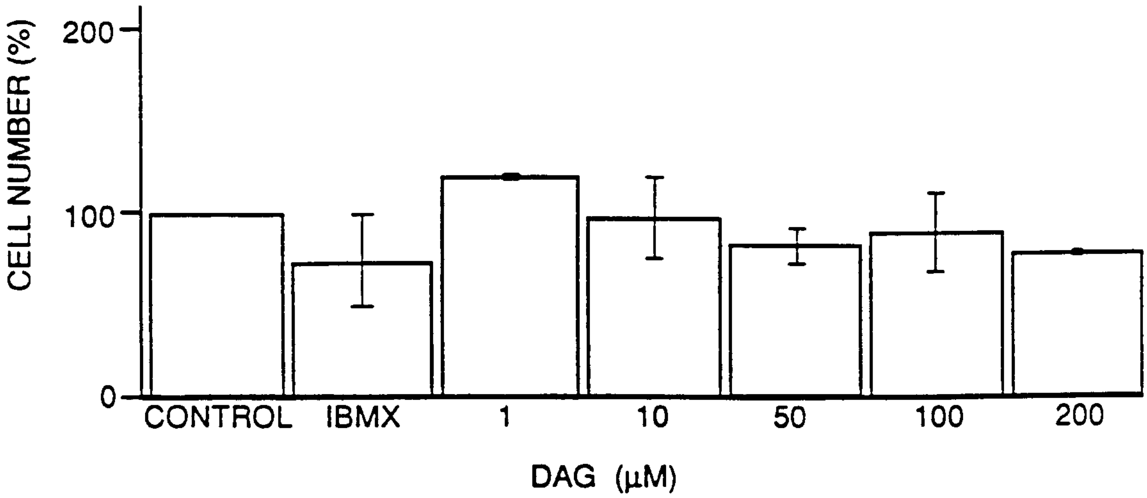
CELL NUMBER (%)
200
100
0
CONTROL
IBMX
1
10
50
100
200
DAG (µM)

FIG 4

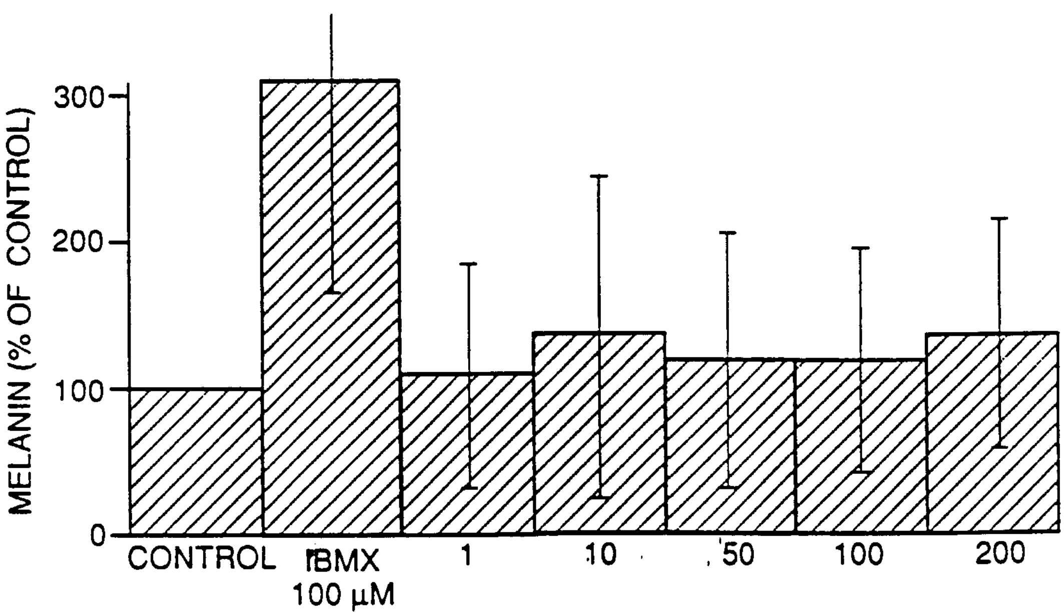
MELANIN (% OF CONTROL)
300
200
100
0
CONTROL
IBMX
100 µM
1
10
50
100
200

FIG 5